(19) Europäisches Patentamt / European Patent Office / Office européen des brevets

(11) **EP 2 759 258 B1**

(12) **EUROPEAN PATENT SPECIFICATION**

(45) Date of publication and mention
of the grant of the patent:
**14.09.2016 Bulletin 2016/37**

(51) Int Cl.:
**A61B 5/021** *(2006.01)*

(21) Application number: **13000376.7**

(22) Date of filing: **25.01.2013**

(54) **Method of approximating a patient's pulse wave based on non-invasive blood pressure measurement, a logic unit therefore and a system therefore**

Verfahren zur Annäherung der Pulswelle eines Patienten basierend auf nichtinvasiver Blutdruckmessung, logische Einheit und System dafür

Procédé d'approximation d'une onde d'impulsion du patient sur la base de la mesure de la pression sanguine non invasive, unité logique associée et système correspondant

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.07.2014 Bulletin 2014/31**

(73) Proprietor: **UP-MED GmbH**
**81673 München (DE)**

(72) Inventors:
• **Knoll, Reinhold**
**94127 Neuburg (DE)**
• **Pfeiffer, Ulrich**
**81667 München (DE)**

(74) Representative: **Ter Meer Steinmeister & Partner Patentanwälte mbB**
**Nymphenburger Straße 4**
**80335 München (DE)**

(56) References cited:
**US-A1- 2003 233 204      US-A1- 2004 002 659**
**US-A1- 2012 078 123      US-B1- 6 503 206**

EP 2 759 258 B1

**Description**

The present invention relates to a method of approximating a patient's pulse wave based on non-invasive blood pressure measurement. The invention also relates to a logic unit and a corresponding system for approximating a patient's pulse wave based on non-invasive blood pressure measurement.

A skilled practitioner, such as an experienced physician, can obtain useful information as to the health status of a patient from an analysis of the curve progression of the arterial blood pressure, i.e. pulse wave, of the patient. The pulse wave of the patient may be reliably measured in an invasive way, by introducing a catheter into one of the patient's blood vessels. However, invasive blood pressure measurement approaches are relatively complex, sometimes being accompanied by adverse side-effects for the patient, such as thrombo-embolic complications, bleedings and infections.

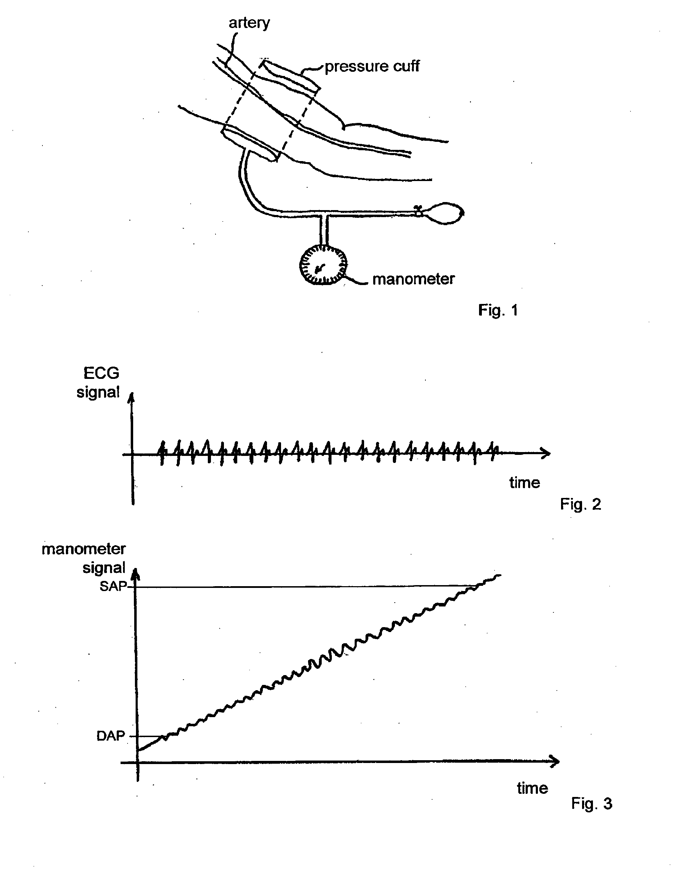

A well-known, less dangerous and more convenient way to determine the arterial blood pressure values of a patient is to use the so-called "oscillometric non-invasive blood pressure measurement method". By that method, a pressure cuff is applied to one of the patient's extremities, preferably to his upper arm at the level of his heart, as schematically illustrated in figure 1. Then, the pressure in the pressure cuff is increased or decreased, usually at a constant rate, thereby exerting pressure on an artery in the patient's extremity. For example, the pressure in the pressure cuff may be increased from a value equal to or smaller than the diastolic arterial blood pressure $DAP$ to a value equal to or greater than the systolic arterial blood pressure $SAP$ of the patient. That is, the pressure in the pressure cuff is continuously increased over a time period which corresponds to a plurality of heart beats.

Figure 2 schematically illustrates an electrocardiogram signal (ECG-signal) over the time. The manometer connected to the pressure cuff (shown in figure 1) not only indicates the continuously increasing pressure applied to the pressure cuff, but in addition (due to the principle action = reaction) also the pulses, as schematically illustrated in figure 3. In the following, the term "pulse" refers to pressure oscillations caused by one heart beat of the patient.

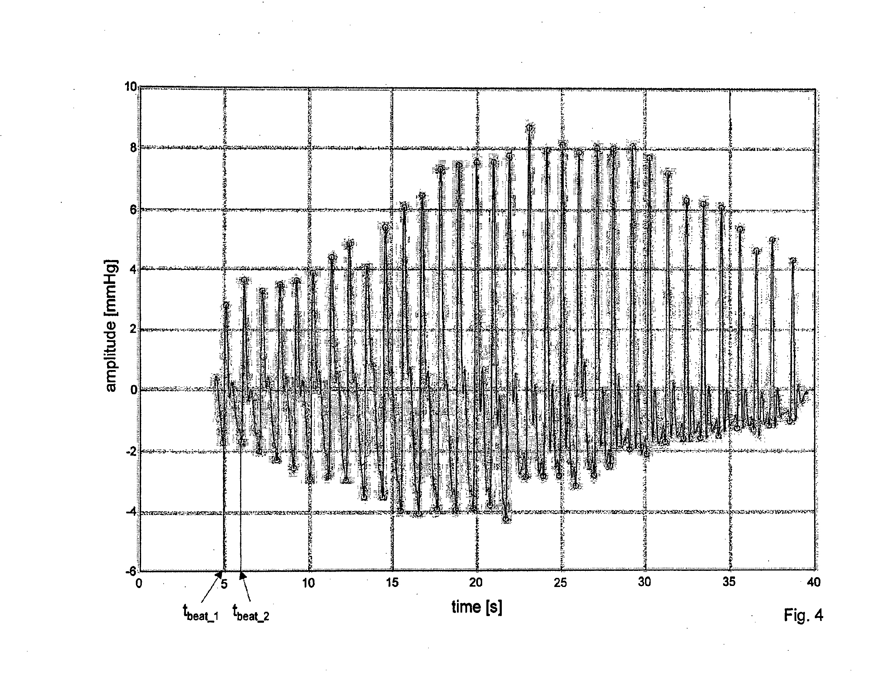

Figure 4 is an exemplary diagram showing exclusively the pulses (i.e. pressure oscillations caused by the patient's heart beats) indicated by the manometer over the time (the pressure variation caused by the continuously increasing cuff pressure is omitted in this diagram). As shown in this diagram, a sequence of pulse signals (caused by a corresponding number of heart beats of the patient) has been measured. The pressure oscillations shown in figure 4 are plotted in such a way that the curve cyclically oscillates around a zero pressure line (i.e. zero pressure value). The area enclosed by the curve below the zero pressure line substantially corresponds to the area enclosed by the curve above the zero pressure line. Small circles in figure 4 indicate the lower and upper extreme values associated to the individual heart beats. That is, a single heart beat of the patient causes a pulse lasting from a first lower extreme value of the curve to a subsequent, second lower extreme value of the curve. This way of representation of a sequence of pulse signals measured by the so-called "oscillometric non-invasive blood pressure measurement method", and how to determine the lower and upper extreme values associated to the individual heart beats, is well known to those skilled in the art.

The distance between two subsequent lower (or upper) extreme values of the curve shown in figure 4 is substantially constant (corresponding to the patient's heart rate). However, the amplitude and the general shape of the measured pulse signals associated to the individual heart beats significantly differ from each other (even though, the actual pulse waves of the patient remain substantially unchanged over the detection time). For example, the amplitude of the measured pulse signals associated to the individual heart beats is not constant, but the curve shown in figure 4 is rather bell-shaped. Furthermore, the pulse signals measured at higher cuff pressures are more ragged than those measured at lower cuff pressures. This phenomenon is characteristic for pulse signals measured by the so called "oscillometric non-invasive blood pressure measurement method".

The above described oscillometric non-invasive blood pressure measurement method is relatively popular, because it enables a skilled practitioner to easily determine the systolic arterial blood pressure $SAP$ and the diastolic arterial blood pressure $DAP$ of a patient (by using an empirical approach). It is known that the oscillation amplitude is between 45-57%, usually 50%, of the maximum oscillation amplitude at a clamp pressure equal to the systolic arterial blood pressure $SAP$, whereas the oscillation amplitude is between 75-86%, usually 80%, of the maximum oscillation amplitude at a clamp pressure equal to the diastolic arterial blood pressure $DAP$. Thus, the absolute pressure values indicated by the manometer at corresponding moments correspond to the diastolic arterial blood pressure $DAP$ and the systolic arterial blood pressure $SAP$. Notably, instead of a classic manometer, an electrical sensor may be equally applied. The above-described principle is conferrable to other physical values, such as acceleration, sound and optical reflection.

Disadvantageously, it is impossible with this known blood pressure measurement method to reliably determine the shape of the pulse wave of the patient's arterial blood pressure. As mentioned above, the exact shape of the patient's pulse wave, however, can provide important information (as to the health status of this patient) to a skilled practitioner, such as an experienced physician.

US 6,503,206 B1 describes an apparatus for monitoring one or more vital signs (e.g., the blood pressure) of a subject. The apparatus comprises a plurality of sensors which are placed at spaced-apart locations on a body and which transmit signals. A selection system of the apparatus determines performance criteria for a number of groups of signals. The apparatus computes a predicted value for a vital sign by either computing a value from each group of signals and taking a weighted average with weights based upon the performance criterion or by selecting one of the groups of signals for which the performance criterion is best and computing the output value from that group of signals.

US 2004/0002659 A1 describes an electronic blood pressure monitor that is configured to calculate an average blood pressure value on the basis of calculated wave feature values of pulse waves detected by a pulse wave detector. For this purpose, pulse waves at different cuff pressures are detected and a calculation of average blood pressure values at the different cuff pressures is performed. Thereafter, the average blood pressure values for each pulse wave are weighted and an average blood pressure value is calculated therefrom.

EP 0 078 090 A1 describes a non-invasive blood pressure measurement method that is - at least theoretically - capable of determining the arterial pulse wave of a patient. According to this method, a fluid-filled pressure cuff is attached to a patient's finger. A light source and a light detector are integrated in the pressure cuff, the light source and the light detector forming part of a photo-electric plethysmograph. The cuff pressure is controlled - via a fast-acting electric pressure valve - in closed-loop operation based on the plethymographic signal, so that the arterial volume in the finger is maintained at a predefined value. Measuring the pressure in the pressure cuff, thus, allows for determining the arterial blood pressure of the patient. This method is also known in literature as "volume-clamp-method".

However, permanently correcting or re-adjusting the pressure in the pressure cuff in real time is technically difficult and prone to errors. Furthermore, till now, this method only works with a pressure cuff applied to a patient's finger. The finger is yet located relatively remote from the patient's heart, so that the diameter of arterial vessels in the finger is relatively small compared to the diameter of arterial vessels close to the heart. Due to interference effects caused by pressure reflections occurring when the diameter of arterial vessels (abruptly) changes, e.g. when arterial vessels branch, the blood pressure measurable at the finger only imprecisely corresponds to the arterial pulse wave at the patient's heart. To consider these interference effects, it was tried to reconstruct the pulse curve in the patient's aorta from the signals measured at the patient's finger, using transfer functions that are usually based on empirical and statistical parameters. However, since the parameters are not (or not sufficiently) adapted to the individual patient and situation, such an approach is not promising, potentially providing imprecise results.

It is therefore the object of the present invention to provide a method and a corresponding device for better approximating a patient's central arterial pulse wave based on non-invasive blood pressure measurement.

This object is achieved by the subject-matters of the independent claims. Preferred embodiments are the subject-matter of the dependent claims.

According to a first aspect of the present invention, there is provided a method of approximating a patient's pulse wave based on non-invasive blood pressure measurement, comprising the following steps:

(a)    non-invasively measuring a sequence $n=1...N$ of pulse signals $pulse_{n\_measured}(t)$ of a patient, thereby applying a clamp pressure $clamp_n(t)$;

(b)    weighting the measured pulse signals $pulse_{n\_measured}(t)$ using a weighting function to obtain weighted pulse signals $pulse_{n\_weighted}(t)$;

(c)    adding up the weighted pulse signals $pulse_{n\_weighted}(t)$ to obtain an approximation of the patient's pulse wave $pulse_{approx}(t)$.

A good approximation of the patient's pulse wave $pulse_{approx}(t)$ can be obtained simply by weighting a corresponding number of measured pulse signals $pulse_{n\_measured}(t)$ and then adding up the weighted pulse signals $pulse_{n\_weighted}(t)$. The method according to the present invention allows - without difficulty - for measuring the pulse signals close to the patient's heart, e.g. at the patient's upper arm, so as to substantially avoid interference effects otherwise occurring when measuring the pulse signals at locations remote from the patient's heart, e.g. at a finger of the patient, as with the above-described volume-clamp-method. Furthermore, the method according to the present invention allows for determining an approximation of the patient's pulse wave without the need of permanently correcting or re-adjusting the pressure in the pressure cuff, like with the volume-clamp-method.

In method step (a) of the inventive method, a sequence $n=1...N$ of pulse signals $pulse_{n\_measured}(t)$ of a patient is measured in an non-invasive fashion, using a (not-constant) clamp pressure $clamp_n(t)$. $N$ corresponds to the total number of individual heart beats of the patient within the detection period. In the exemplary diagram shown in figure 4, about 32 "pulse waves" corresponding to 32 individual heart beats have been measured within the detection period. Therefore, in this example, $N=32$ and, thus, $n=1...32$.

Preferably, the well-known and relatively comfortable "oscillometric non-invasive blood pressure measurement method" (as described above) is applied to measure the patient's pulse signals. In this method, the clamp pressure $clamp_n(t)$ is applied to an extremity of the patient, preferably to an upper arm of the patient, as shown e.g. in figure 1. The clamp pressure ($clamp_n(t)$) is preferably varied between a value equal to or smaller than the diastolic arterial blood pressure $DAP$ and a value equal to or greater than the systolic arterial blood pressure $SAP$ of the patient. As known in the art, for this purpose SAP and DAP is estimated or derived from previous measurements.

Thereby, the clamp pressure $clamp_n(t)$ may be increased or decreased continuously, preferably at a substantially constant rate. Notably, the increase or decrease rate should be low enough so as to detect a sufficient number of pulses caused by individual heart beats of the patient (preferably at least 10). The clamp pressure $clamp_n(t)$ may be continuously increased or decreased between the diastolic arterial blood pressure $DAP$ and the systolic arterial blood pressure $SAP$ within a detection period of e.g. about one minute. During this detection period, pulse signals $pulse_{n\_measured}(t)$ associated to e.g. 60 individual heart beats of the patient may be measured, which represents a very good base for the further method steps. However, to avoid problems caused by blockage of blood circulation in the patient's extremity to which the pressure cuff is applied, the increase or decrease rate of the clamp pressure $clamp_n(t)$ should not be too low, i.e. the detection time should preferably not exceed one minute.

If the increase or decrease rate of the clamp pressure $clamp_n(t)$ is moderate (e.g. the detection time is about one minute), the clamp pressure $clamp_n(t)$ associated with the time period of one individual heart beat might be considered - for the sake of simplicity - as being substantially constant. For example, the clamp pressure $clamp_n(t)$ associated with the time period of one individual heart beat might be approximated so as to correspond to the actual clamp pressure at the beginning ($t=t_{beat\_n}$) of the corresponding heart beat ($clamp_n = clamp(t_{beat\_n})$). In the example shown in figure 4, the clamp pressure $clamp_n(t)$ associated with the first detected heart beat ($n=1$) thus corresponds to the clamp pressure at the time beginning of the first heart beat at $t=t_{beat\_1}=5s$, i.e. $clamp_1=clamp(t_{beat\_1})$. Accordingly, the clamp pressure $clamp_n(t)$ associated with the second detected heart beat ($n=2$) corresponds to the clamp pressure at the beginning of the second heart beat at $t=t_{beat\_2}=6s$, i.e. $clamp_2=clamp(t_{beat\_2})$.

However, the clamp pressure $clamp_n(t)$ associated with the time period of one individual heart beat might equally be approximated so as to correspond e.g. to the actual clamp pressure at the end or somewhere in the middle (preferably exactly in the middle) of the corresponding heart beat.

Notably, if the sequence of pulse signals $pulse_{n\_measured}(t)$ has been previously measured and stored, the method step (a) might be skipped and the method according to the present invention may directly start with method step (b) based on the previously stored signal values.

In method step (b) of the inventive method, the measured pulse signals $pulse_{n\_measured}(t)$ are weighted to obtain weighted pulse signals $pulse_{n\_weighted}(t)$. A weighting function is applied in method step (b), as will be described in more detail below.

Finally, in method step (c), the weighted pulse signals $pulse_{n\_weighted}(t)$ are added up to obtain the approximation of the patient's pulse wave $pulse_{approx}(t)$. In method step (c), the approximation of the patient's pulse wave $pulse_{approx}(t)$ might be simply calculated as follows:

$$pulse_{approx}(t) = \sum_{n=1}^{N} pulse_{n\_weighted}(t).$$

As set forth above, the measured pulse signals $pulse_{n\_measured}(t)$, i.e. the cyclic pressure variations corresponding to the individual heart beats, detected by the manometer (as shown in figure 4), not only vary with respect to their amplitude, but they are also significantly distorted as to the shape of the pulse waves. Even though, this phenomenon is not yet completely understood, it is supposed that it is mainly caused by the non-resilience of the body tissue between the artery and the pressure cuff.

The inventors have found out that at moments, when the actual internal pressure (i.e. arterial blood pressure) equals a predetermined difference, e.g. approximately zero, to the externally applied pressure (i.e. cuff pressure), there exists a substantially linear relationship between the measured pulse signals and the actual arterial blood pressure (for example, at moments when the applied cuff pressure substantially equals the actual internal arterial blood pressure, the body tissue between the artery, e.g. in the upper arm, and the pressure cuff is relaxed, i.e. not biased).

Therefore, it is advantageous - in order to obtain an improved approximation of the patient's pulse wave - to use the clamp pressure $clamp_n(t)$ as an input parameter of the weighting function, wherein the weighting function is preferably a differential pressure function. That is, the measured pulse signals $pulse_{n\_measured}(t)$ may be weighted in such a way that those portions of the curve of the measured pulse signals $pulse_{n\_measured}(t)$ are more "emphasised" that have been measured during moments at which the actual internal arterial blood pressure equals a predetermined percentage of the externally applied cuff pressure.

However, a problem resides in that usually the moments, at which the actual internal arterial blood pressure equals a predetermined percentage of the externally applied cuff pressure, are unknown, because the actual internal arterial blood pressure (i.e. the patient's pulse wave) is unknown. In fact, an approximation of the patient's pulse wave is the pursued result of the method.

To overcome this problem, method steps (b) and (c) of the method according to the present invention are preferably iteratively repeated at least one more time. The outcome of the first iteration loop, i.e. the approximation of the patient's pulse wave, can then be used as approximation of the actual internal arterial blood pressure in the second iteration loop. Thus, the moments, at which the actual internal arterial blood pressure equals a predetermined percentage of the externally applied cuff pressure, can be (at least approximately) determined. In the second iteration loop, the measured pulse signals $pulse_{n\_measured}(t)$ can then be weighted accordingly (in step (b) of the second iteration loop) before adding up the weighted pulse signals $pulse_{n\_weighted}(t)$ (in step (c) of the second iteration loop) so as to obtain an improved approximation of the patient's pulse wave $pulse_{approx}(t)$.

The outcome of the method can be even further improved by iteratively repeating method steps (b) and (c) more than two times, wherein the outcome of method step (c) of the previously iteration loop is used as input value for the present iteration loop. Preferably, the weighting function of the present iteration loop is a differential pressure function comprising, as an input parameter, the externally applied cuff pressure and, as another input parameter, the approximation of the actual internal arterial blood pressure, i.e. the approximated patient's pulse wave $pulse_{approx}(t)$ determined in the previous iteration loop.

Of course, it is not possible to determine the weighting function to be applied in the very first iteration loop that way, since there is no result of a previous iteration loop available as input. Consequently, the weighting function applied in the first iteration loop preferably differs from the weighting function applied in the second and/or higher iteration loop. The first iteration loop, thus, provides a more roughly approximated pulse wave $pulse_{approx}(t)$ of the patient compared to following iteration loops. For example, the weighting function of the first iteration loop might simply be determined as follows:

$$weight1_n = 1 \quad \text{if} \quad DAP < clamp_n(t) < SAP,$$

$$weight1_n = 0 \quad \text{otherwise,}$$

wherein *DAP* corresponds to the diastolic arterial blood pressure and *SAP* corresponds to the systolic arterial blood pressure of the patient.

In such a case, in method step (c) of the first iteration loop, the following formula might be applied for calculating the approximated pulse wave $pulse_{approx}(t)$:

$$pulse_{approx}(t) = \frac{1}{N} \sum_{n=1}^{N} pulse_{n\_weighted}(t) \times weight1_n \, .$$

As mentioned above, the weighting function applied in the second and/or higher iteration loop is preferably a differential pressure function having the clamp pressure $clamp_n(t)$ as an input parameter and having the approximated pulse wave $pulse_{approx}(t)$ obtained in the previous iteration loop as another input parameter.

For example, the weighting function applied in the second and/or higher iteration loop can be a triangular function, preferably having its maximum when the clamp pressure $clamp_n(t)$ equals a predetermined difference, preferably zero, to the approximated pulse wave $pulse_{approx}(t)$ obtained in a previous iteration loop.

If a triangular function is applied as weighting function $weight_n(t)$ in the second and/or higher iteration loop, the weighting function $weight_n(t)$ might be calculated as follows:

$$weight_n(t) = 1 - \frac{clamp_n - pulse_{approx\_prev}(t)}{clamp_n - clamp_{n-1}} \quad \text{if} \quad clamp_{n-1} < pulse_{approx\_prev}(t) < clamp_n;$$

$$weight_n(t) = 1 - \frac{pulse_{approx\_prev}(t) - clamp_n}{clamp_{n+1} - clamp_n} \quad \text{if} \quad clamp_n \le pulse_{approx\_prev} < clamp_{n+1}; \text{ and}$$

$$weight_n(t) = 0 \quad \text{otherwise.}$$

As mentioned above, the index *n* refers to the number of the heart beat of the corresponding measured pulse signal $pulse_{n\_measured}(t)$. As mentioned above, even though, the clamp pressure $clamp_n$ may not be constant over the detection period of one pulse wave, for the sake of simplicity, the clamp pressure $clamp_n$ might be considered as being substantially constant during this detection period, e.g. corresponding to the clamp pressure $clamp_n = clamp_n(t=t_{beat\_n})$ at the beginning of the corresponding detection period. $pulse_{approx\_prev}(t)$ corresponds to the result, i.e. the approximated pulse wave of the patient, of the previous iteration loop.

As an alternative to a triangular weighting function, the weighting function applied in the second and/or higher iteration loop may be a bell-shaped function, preferably having its maximum when the clamp pressure $clamp_n(t)$ equals a predetermined difference, preferably zero, to the approximated pulse wave $pulse_{approx}(t)$ obtained in a previous iteration loop.

When using a bell-shaped function as weighting function $weight_{approx\_n}(t)$ in the second and/or higher iteration loop, the weighting function $weight_n(t)$ might be calculated as follows:

$$weight_n(t) = \frac{1}{1 + \left( \dfrac{clamp_n - pulse_{approx\_prev}(t)}{p_w} \right)^2}$$

wherein parameter $p_w$ corresponds to an empirically determined parameter being decisive for the width at half maximum of the bell-shaped weighting function. The parameter $p_w$ is preferably chosen in accordance with the particular circumstances of the blood pressure measurement that have an influence on the distortion of the measured pulse curves. If distortion of the measured pulse curves increases (e.g. due to the use of another blood pressure measurement device), the increase or decrease rate of the cuff pressure should be decreased so as to measure more pulses of the patient within the detection time. In such a case, a smaller value for the parameter $p_w$ may be chosen. Generally, the parameter $p_w$ may preferably be chosen according to the following equation:

$$p_w = \frac{SAP - DAP}{N},$$

wherein $N$ is the total number of pulses measured during the detection period, i.e. measured substantially during the time needed by the cuff pressure to change from the diastolic arterial blood pressure $DAP$ to the systolic arterial blood pressure $SAP$ of the patient, or the other way around.

Preferably, method step (c) of the second and/or higher iteration loop further comprises: scaling the approximated pulse wave $pulse_{approx}(t)$ to the difference between the diastolic blood pressure value $DAP$ and the systolic blood pressure value $SAP$ of the patient. An example of such a scaling is provided below.

Scaling the approximated pulse wave $pulse_{approx}(t)$ ensures that the amplitude of the (scaled) approximated pulse wave correctly corresponds to the amplitude of the actual pulse wave of the patient. That is, the approximated pulse wave $pulse_{approx}(t)$ is scaled in such a way that its lower extreme value substantially corresponds to the diastolic blood pressure value $DAP$ of the patient, whereas its upper extreme value substantially corresponds to the systolic blood pressure value $SAP$ of the patient. As mentioned before, it is well-known to those skilled in the art, how to determine diastolic and systolic arterial blood pressure values $DAP$ and $SAP$ based on the so-called "oscillometric non-invasive blood pressure measurement method".

If the approximated pulse wave $pulse_{approx}(t)$ is scaled in method step (c) of the second and/or higher iteration loop, the scaled approximated pulse wave $pulse_{approx\_scaled}(t)$ (instead of the approximated pulse wave $pulse_{approx}(t)$) is applied in method step (b) of the subsequent iteration loop.

Similarly, method step (a) may further comprise: scaling the measured pulse signals $pulse_{n\_measured}(t)$ to the difference between the diastolic blood pressure value $DAP$ and the systolic blood pressure value $SAP$ of the patient.

Scaling of the measured pulse signals $pulse_{n\_measured}(t)$ in method step (a) might be performed by applying the following formula:

$$pulse_{n\_measured\_scaled}(t) = offset_n + scale_n \times pulse_{n\_measured}(t),$$

wherein the parameter $offset_n$ is preferably calculated as follows:

$$offset_n = DAP - \min\left(pulse_{n\_measured}(t)\right), \text{ and}$$

wherein the parameter $scale_n$ is preferably calculated as follows:

$$scale_n = \frac{SAP - DAP}{\max\left(pulse_{n\_measured}(t)\right) - \min\left(pulse_{n\_measured}(t)\right)}.$$

$\max(pulse_{n\_measured}(t))$ corresponds the maximum value of the measured pulse wave corresponding to the heart beat with the number $n$. Similarly, $\min(pulse_{n\_measured}(t))$ corresponds the minimum value of the measured pulse wave corresponding to the heart beat with the number $n$.

Of course, as will be apparent to those skilled in the art, other formulas may be applied to calculate the parameters $offset_n$ and $scale_n$. For example the formulas might equally be based on the mean arterial pressure $MAP$ of the patient, which can also be determined based on the so-called "oscillometric non-invasive blood pressure measurement method".

If the measured pulse signals $pulse_{n\_measured}(t)$ are scaled in method step (a), the scaled measured pulse signals $pulse_{n\_measured}(t)$ (instead of the measured pulse signals $pulse_{n\_measured}(t)$) are applied in method step (b) to determine the weighted pulse signals $pulse_{n\_weighted}(t)$.

In such a case, in method step (c) of the first iteration loop, the following formula might be applied for calculating the approximated pulse wave $pulse_{approx}(t)$:

$$pulse_{approx}(t) = \frac{1}{\displaystyle\sum_{n=1}^{N} weight_n(t)} \sum_{n=1}^{N} pulse_{n\_measured\_scaled}(t) \times weight_n(t),$$

wherein the weighting function $weight_n(t)$ applied in the first iteration loop is preferably a function of a difference between scaled measured pulse signals $pulse_{n\_measured\_scaled}(t)$ and the clamp pressure $clamp_n(t)$.

According to another aspect, the invention refers to a logic unit for approximating a patient's pulse wave based on a non-invasive blood pressure measurement, configured to carry out the following steps:

- weighting previously measured pulse signals $pulse_{n\_measured}(t)$ using a weighting function to obtain weighted pulse signals $pulse_{n\_weighted}(t)$;
- adding up the weighted pulse signals $pulse_{n\_weighted}(t)$ to obtain an approximation of the patient's pulse wave $pulse_{approx}(t)$,

wherein these steps are preferably iteratively repeated at least one more time.

The logic unit according to the present invention is configured to carry out the above described method, wherein the sequence of pulse signals $pulse_{n\_measured}(t)$ has been previously measured and stored, so that method step (a) can be skipped and the logic unit according to the present invention directly starts with method step (b), based on the previously stored signal values.

According to yet another aspect, the present invention also refers to a system for approximating a patient's pulse wave based on a non-invasive blood pressure measurement, comprising the logic unit described above and a blood pressure measurement device, the blood pressure measurement device being configured for non-invasively measuring a sequence $n=1...N$ of pulse signals of a patient to obtain measured pulse signals $pulse_{n\_measured}(t)$, wherein the system is configured for providing the measured pulse signals $pulse_{n\_measured}(t)$ as input values to the logic unit. Thus, the system is also configured to obtain the measured pulse signals $pulse_{n\_measured}(t)$ according to step (a) of the above described method.

Preferably, the blood pressure measurement device comprises a pressure cuff, and even more preferably, the pressure cuff is configured for being disposed around a patient's arm so as to measure the patient's arterial blood pressure in a non-invasive way. Thus, the system is configured to obtain the measured pulse signals $pulse_{n\_measured}(t)$ using the above-described "oscillometric non-invasive blood pressure measurement method". Since the pressure cuff is configured for being attached around a patient's arm, preferably an upper arm of the patient, substantially no interference effects caused by pressure reflections adversely affect the measurement - contrary to the above described "volume-clamp-method".

Even though, the "oscillometric non-invasive blood pressure measurement method" exhibits the advantage that substantially no interference effects caused by pressure reflections adversely affect the measurement (in contrast to known methods for measuring peripheral blood pressure waveform data, such as the above described "volume-clamp-method" utilized on a finger or the so called "applanation-tonometry-method" utilized at the patient's wrist), the "oscillometric non-invasive blood pressure measurement method" does not allow for continuous measurements without blocking blood flow in an unallowable manner. However, continuous measurement can be performed with the "volume-clamp-method" or with the "applanation-tonometry-method".

As described above, it has already been tried in the past to overcome the disadvantages of the known methods for measuring peripheral blood pressure waveform data, such as the "volume-clamp-method" and the "applanation-tonometry-method", by using transfer functions in order to reconstruct the central blood pressure waveforms from peripherally measured signals, e.g. signals measured at a patient's finger. However, since the applied transfer functions are usually based on statistical and empirical parameters that are not (or at least not sufficiently) adapted to the individual patient and situation, such an approach bears the likelihood to provide imprecise results.

Using pulse signals measured e.g. with the above described "oscillometric non-invasive blood pressure measurement method" to calibrate the transfer function to an individual patient did not represent a promising approach, either, since, in the past, it was not possible to approximate (with sufficient quality) the patient's pulse wave based on the measured pulse signals. However, with the method according to the present invention, an approximation of good quality of the patient's pulse wave based on non-invasive blood pressure measurement becomes possible. Therefore, the system described above may be combined with a device for measuring peripheral blood pressure waveform data, and a transfer function may be applied, wherein the transfer function is calibrated to an individual patient based on the patient's pulse wave that has been previously approximated according to the method of the present invention. That way, the central arterial blood pressure waveforms can be continuously determined with high quality.

Thus, the previously described system for approximating a patient's pulse wave based on a non-invasive blood pressure measurement preferably further comprises a second blood pressure measurement device that is adapted for non-invasively measuring peripheral blood pressure waveform data of the patient in a continuous way, wherein the system is adapted to apply a transfer function to reconstruct central blood pressure waveforms from the measured peripheral blood pressure waveform data based on the approximated pulse wave $pulse_{approx}(t)$.

The patient's pulse wave may be approximated according to the inventive method only once, preferably just before the continuous measurement of the peripheral blood pressure waveform data.

More preferably, the approximated pulse wave $pulse_{approx}(t)$ of the patient is yet determined at substantially regular intervals, wherein the transfer function is regularly recalibrated based on the regularly determined approximated pulse wave $pulse_{approx}(t)$ of the patient. For example, the pulse wave may be approximated according to the method of the present invention every two minutes. This way, it is possible to continuously obtain central blood pressure waveforms of very good quality.

For example, applying the transfer function may comprise the following steps: In a first step, both time-varying signals, i.e. the intermittent determined approximated pulse waves $pulse_{approx}(t)$ and the continuously measured peripheral blood pressure waveforms, are transformed into the frequency domain. Then, in a second step, the transfer function is determined. In a third step, the transfer function is applied to the peripherally measured blood pressure waveforms so as to calibrate the peripheral blood pressure waveforms. Finally, in a fourth step, the calibrated peripheral blood pressure waveforms are re-transformed into the time domain.

Exemplary embodiments of the present invention are described in more detail below based on the figures, in which:

figure 1: schematically shows a known pressure cuff configuration used to carry out the so-called "oscillometric non-invasive blood pressure measurement method";

figure 2: schematically illustrates an electrocardiogram signal (ECG-signal) over the time, which signal has been measured with the pressure cuff configuration shown in figure 1;

figure 3: schematically illustrates the signal of the manometer of the pressure cuff configuration shown in figure 1 over the time;

figure 4: represents an exemplary diagram showing exclusively the pulses, i.e. pressure oscillations caused by the patient's heart beats, indicated by the manometer over the time, thereby omitting the pressure variation caused by the continuously increasing cuff pressure;

figure 5: illustrates an exemplary diagram showing approximated pulse waves of the patient, determined according to the method of the present invention;

figure 6: illustrates the functioning of a first iteration loop of the method of the present invention;

figure 7: illustrates the functioning of a second and/or higher iteration loop of the method of the present invention;

figure 8: illustrates a block diagram for continuously obtaining central blood pressure waveforms of high good quality.

As described above, figures 1-4 all refer to a well-known method of non-invasively measuring blood pressure signals and to a conventional way of processing and representing the measured signals. In particular, figure 1 shows a known pressure cuff configuration comprising a manometer and used to carry out the so-called "oscillometric non-invasive blood pressure measurement method". Furthermore, figure 4 is an exemplary diagram showing exclusively the pulses, i.e. pressure oscillations caused by the patient's heart beats, indicated by the manometer over the time, whereas the pressure variation caused by the continuously increasing cuff pressure is omitted in this diagram. As shown in figure 4, a sequence of pulse signals (caused by a corresponding number of heart beats of the patient) has been measured. The pressure oscillations shown in figure 4 are plotted in such a way that the curve cyclically oscillates around a zero pressure line (or zero pressure value). How to determine such a diagram is well known to those skilled in the art.

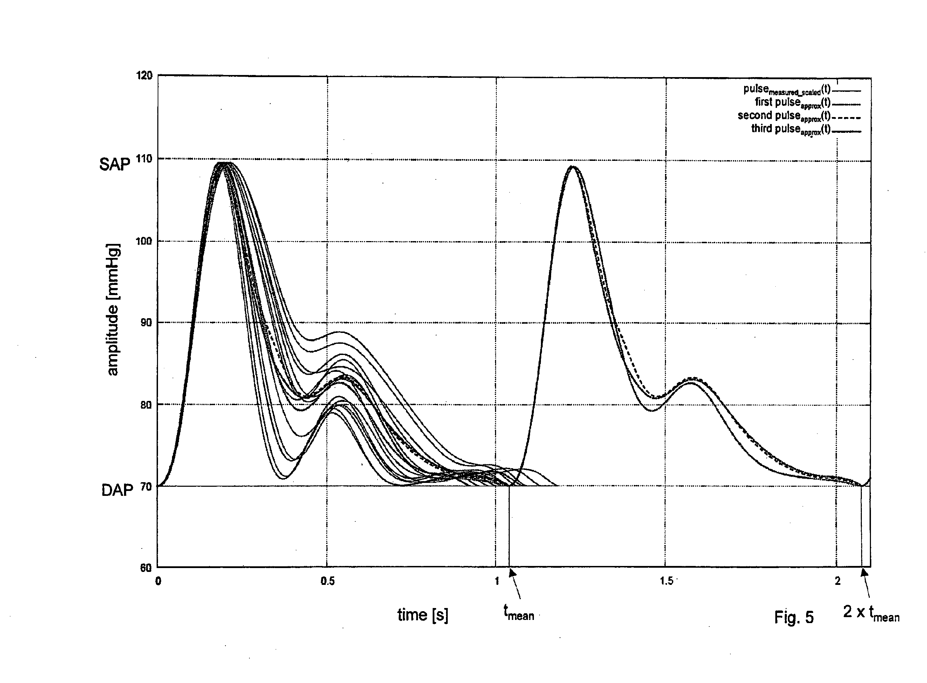

In the following, the present invention will be described in more detail in view of figures 5-8.

Figure 5 shows a diagram with time (in seconds) on its axis of abscissas and with pressure (in mmHg) on its axis of ordinates. Furthermore, a plurality (13 in this example, i.e. $N=13$ and $n=1...13$) of measured and scaled pulse signals $pulse_{n\_measured}(t)$ of a patient are represented in the diagram. In this example, the axis of abscissas corresponds to $t-t_{beat\_n}$ (as previously mentioned $t_{beat\_n}$ corresponds to the moment of beginning of a heart beat) so that the illustrated 13 curves $pulse_{n\_measured\_scaled}(t)$ all start at 0 seconds with the same value, namely with the diastolic blood pressure value $DAP$ of the patient.

The measured pulse signals $pulse_{n\_measured}(t)$ of a patient are scaled so as to make the amplitudes of the (scaled) measured pulse signals all correspond to the amplitude of the actual pulse wave of the patient. That is, the measured pulse signals $pulse_{n\_measured}(t)$ are scaled in such a way that the lower extreme value of each measured pulse signal substantially corresponds to the diastolic blood pressure value $DAP$ of the patient, whereas its upper extreme value substantially corresponds to the systolic blood pressure value $SAP$ of the patient. As mentioned before, it is well-known to those skilled in the art, how to determine diastolic and systolic arterial blood pressure values $DAP$ and $SAP$, e.g. by applying the "oscillometric non-invasive blood pressure measurement method".

Even though the measured and scaled pulse signals $pulse_{n\_measured\_scaled}(t)$ all have the same amplitude, figure 5 clearly shows that they significantly differ from each other with respect to their wave form.

Furthermore, a curve named *"first pulse_{approx}(t)"* is illustrated in figure 5 by a dotted line. This curve has been determined in the first iteration loop of the method according to the invention by simply averaging the curves of the measured and scaled pulse signals $pulse_{n\_measured\_scaled}(t)$. Notably, only those measured pulse signals $pulse_{n\_measured}(t)$ have been taken into account that have been measured with the clamp pressure $clamp_n(t)$ being between the diastolic and the systolic pressure $DAP$ and $SAP$ of the patient.

In other words, the following formula has been applied for calculating the approximated pulse wave *first pulse_{approx}(t)* of the first iteration loop:

$$first\_pulse_{approx}(t) = \frac{1}{N}\sum_{n=1}^{N} pulse_{n\_weighted\_scaled}(t) \times weight1_n \text{ ,with:}$$

$$weight1_n = 1 \quad \text{if} \quad DAP < clamp_n(t) < SAP,$$

$$weight1_n = 0 \quad \text{otherwise.}$$

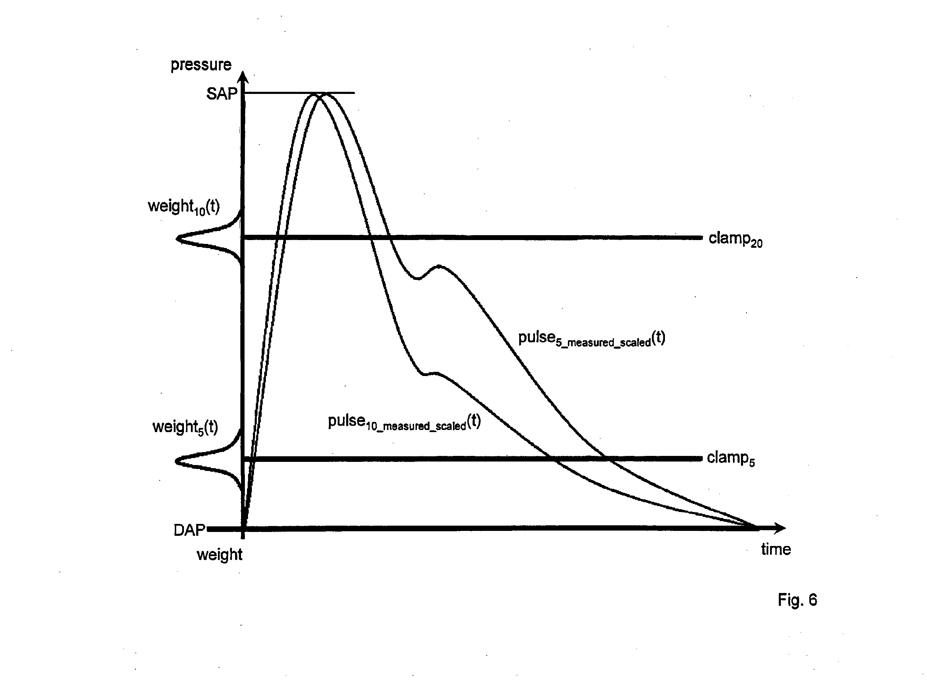

However, instead of obtaining the approximated pulse wave *first pulse_{approx}(t)* of the first iteration loop by simply averaging the curves of the measured and scaled pulse signals $pulse_{n\_measured\_scaled}(t)$, the measured and scaled pulse signals $pulse_{n\_measured\_scaled}(t)$ may be weighted in a more sophisticated way before adding them up. For example, a bell-shaped weighting function may be applied is schematically illustrated in figure 6.

Figure 6 shows a schematic example of only two (for the sake of clarity) measured and scaled pulse signals $pulse_{n\_measured\_scaled}(t)$, namely for the fifth ($n=5$) and the tenth ($n=10$) heart beat. A bell-shaped function is applied as weighting function $weight_n(t)$, so as to particularly accentuate those portions of the scaled measured and scaled pulse signals $pulse_{n\_measured\_scaled}(t)$ that substantially correspond to the clamp pressure associated to the corresponding heart beat ($clamp_n = clamp(t_{beat\_n})$).

Calculation of the bell-shaped weighting function $weight_n(t)$ for the first iteration loop works substantially analogue to the calculation of the bell-shaped weighting function $weight_n(t)$ for the second or higher iteration loop, which has been described in detail above.

Generally, the approximated pulse wave $pulse_{approx}(t)$ lasts on the axis of the abscissas $t-t_{beat\_n}$ from 0 seconds till a mean pulse duration time $t_{mean}$ of the measured and scaled pulse signals $pulse_{n\_measured\_scaled}(t)$. In the example shown in figure 5, the mean pulse duration time $t_{mean}$ is at about 1.05 seconds. For determining the approximated pulse wave $pulse_{approx}(t)$, if the duration of a corresponding pulse of the measured and scaled pulse signals

$pulse_{n\_measured\_scaled}(t)$ is shorter than the mean pulse duration time $t_{mean}$, the latest samples of the measured and scaled pulse signals $pulse_{n\_measured\_scaled}(t)$ of the corresponding pulse are duplicated. On the other hand, if duration of a corresponding pulse of the measured and scaled pulse signals $pulse_{n\_measured\_scaled}(t)$ is longer than the mean pulse duration time $t_{mean}$, the samples after the mean pulse duration time $t_{mean}$ are simply omitted.

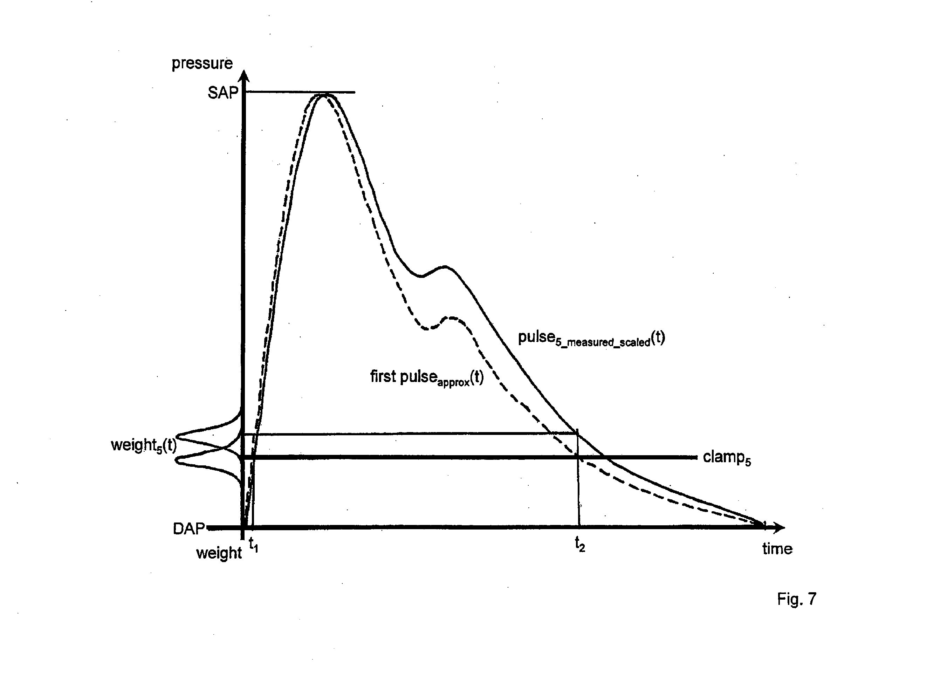

Moreover, figure 5 also exhibits a curve named *"second pulse_{approx}(t)"* illustrated by a dashed line. This curve represents an approximation of the patient's pulse wave obtained in a second iteration loop of the method according to the invention, by applying a bell-shaped weighting function, as will be explained in more detail in view of figure 7.

Figure 7 schematically shows only one (for the sake of clarity) of the measured and scaled pulse signals $pulse_{n\_measured\_scaled}(t)$, namely for the fifth ($n=5$) heart beat. Additionally, figure 5 shows - indicated by a dashed line - the approximated pulse wave *first pulse_{approx}(t)* determined in the first iteration loop. As an approximation, it is assumed that the approximated pulse wave *first pulse_{approx}(t)* determined in the first iteration loop corresponds to the actual pulse wave of the patient. In the present example, the approximated pulse wave *first pulse_{approx}(t)* equals the clamp pressure $clamp_5=clamp(t_{beat\_5})$ two times, namely at moments $t_1$ and $t_2$, as shown in figure 7. At these moments, the fifth measured and scaled pulse signals $pulse_{5\_measured\_scaled}(t)$ is assumed to exhibit a linear relationship with respect to the actual internal arterial blood pressure. Therefore, the fifth measured and scaled pulse signal $pulse_{5\_measured\_scaled}(t)$ is weighted so as to particularly accentuate portions of that curve corresponding to moments $t_1$ and $t_2$. As further shown in figure 7, a bell-shaped function is applied as weighting function $weight_5(t)$ for weighting the fifth measured and scaled pulse signal curve $pulse_{5\_measured\_scaled}(t)$. As can be seen from figure 7, the value of the fifth measured and scaled pulse signal curve $pulse_{5\_measured\_scaled}(t)$ at moment $t_1$ is substantially equal to the value of the weighting function $weight_5(t)$ at moment $t_1$. However, the value of the fifth measured and scaled pulse signal curve $pulse_{5\_measured\_scaled}(t)$ at moment $t_2$ significantly differs from the value of the weighting function $weight_5(t)$ at moment $t_2$.

The same is repeated for all measured and scaled pulse signals $pulse_{n\_measured\_scaled}(t)$, wherein always those portions of the signal curves are particularly accentuated which correspond to moments at which the approximated pulse wave *first pulse_{approx}(t)* determined in the first iteration loop substantially equals the corresponding clamp pressure $clamp_n$. Then, the weighted pulse signals $pulse_{n\_weighted}(t)$ are added up to obtain a better approximation of the patient's pulse wave *second pulse_{approx}(t)* as result of the second iteration loop.

Notably, it is not necessary to accentuate the portions of the signal curves which correspond to moments at which the approximated pulse wave *first pulse_{approx}(t)* determined in the first iteration loop substantially equals the corresponding clamp pressure $clamp_n$ (i.e. the difference is zero). Instead, it is also possible to apply another difference, as long as the same difference is applied for weighting all of the measured and scaled pulse signals $pulse_{n\_measured\_scaled}(t)$.

Finally, figure 5 also exhibits a curve named *"third pulse_{approx}(t)"* illustrated by a chain dotted line. This curve is obtained as a result of the third iteration loop of the method of the present invention in a substantially analogous way as the curve named *"second pulse_{approx}(t)"*. However, instead of applying the curve named *"first pulse_{approx}(t)"* as an approximation of the actual pulse wave of the patient, in the third iteration loop, the curve named *"second pulse_{approx}(t)"* is applied. As can be seen from figure 5, the result of the third iteration loop is already quite similar to the result of the second iteration loop. However, if desired, further iteration loops may be carried out.

It should be noted that in figure 5, only for the sake of clarity, the curve *"first pulse_{approx}(t)"*, the curve *"second pulse_{approx}(t)"*, and the curve *"third pulse_{approx}(t)"* are each shown two times in figure 5, one time lasting on the axis of the abscissas $t-t_{beat\_n}$ from 0 seconds till $t_{mean}$, and (additionally) a second time lasting from $t_{mean}$ till $2 \times t_{mean}$.

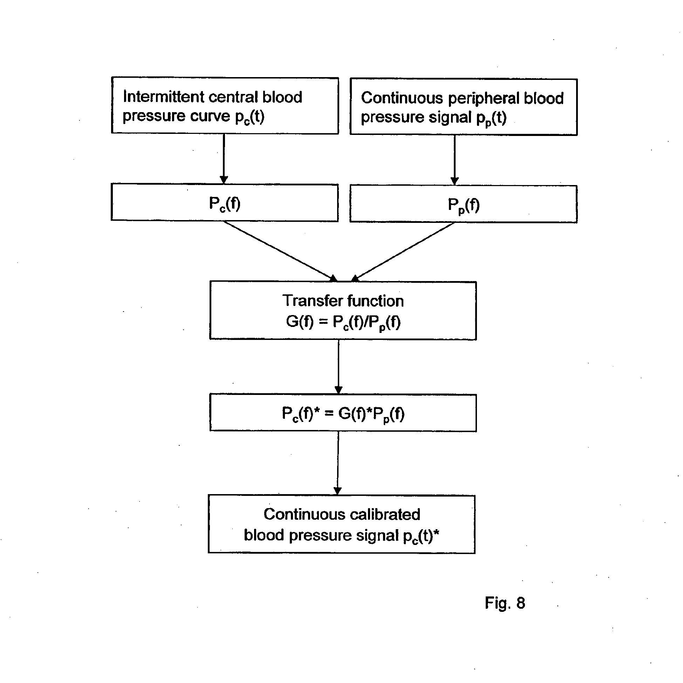

Figure 8 illustrates a block diagram for a method of continuously obtaining central blood pressure waveforms of high quality. This method can be carried out by a system according to the present invention, the system comprising a logic unit capable of performing the method according to the present invention and a first blood pressure measurement device being configured for non-invasively measuring a sequence $n=1...N$ of pulse signals of a patient to obtain measured pulse signals $pulse_{n\_measured}(t)$ as input values for the logic unit. Preferably, the first blood pressure measurement device comprises a pressure cuff adapted for being disposed around a patient's upper arm to measure the patient's arterial blood pressure in a non-invasive way, as shown e.g. in figure 1. The system further comprises a second blood pressure measurement device that is adapted for non-invasively measuring peripheral blood pressure waveform data of the patient in a continuous way. The second blood pressure measurement device is preferably capable to perform the volume-clamp-method, described above.

With such a system, the method shown in figure 8 can be carried out. According to this method, the approximated pulse wave $pulse_{approx}(t)$ of the patient is repeatedly determined according to the method of the present invention, preferably at substantially regular intervals, using the first blood pressure measurement device, thereby obtaining intermittent central arterial blood pressure curves $p_c(t)$. At the same time, peripheral blood pressure signals $p_p(t)$ are contin-uously measured by the second blood pressure measurement device.

E.g. the intermittent central blood pressure curve $p_c(t)$ and the peripheral blood pressure signal $p_p(t)$ are then both transformed into the frequency domain, so as to obtain a central blood pressure signal curve in the frequency domain $P_c(f)$ and a peripheral blood pressure signal curve in the frequency domain $P_p(f)$.

Next, a transfer function G(f) is calculated, based on the central blood pressure signal curve in the frequency domain $P_c$(f) and a peripheral blood pressure signal curve in the frequency domain $P_p$(f).

A calibrated blood pressure signal curve $P_c$(f)* can then be simply obtained by multiplying the transfer function G(f) with the a peripheral blood pressure signal curve in the frequency domain $P_p$(f).

Finally, a calibrated blood pressure curve signal $p_c$(t)* is determined by transforming the calibrated blood pressure signal curve $P_c$(f)* again into the time domain.

**Claims**

1. Method of approximating a patient's pulse wave based on non-invasive blood pressure measurement, comprising the following steps:

   (a) non-invasively measuring a sequence $n=1...N$ of pulse signals $pulse_{n\_measured}$(t) of a patient, thereby applying a not-constant clamp pressure $clamp_n$(t);
   (b) weighting the measured pulse signals $pulse_{n\_measured}$(t) using a weighting function to obtain weighted pulse signals $pulse_{n\_weighted}$(t);
   (c) adding up the weighted pulse signals $pulse_{n\_weighted}$(t) to obtain an approximation of the patient's pulse wave $pulse_{approx}$(t).

2. Method according to claim 1, wherein the clamp pressure $clamp_n$(t) is an input parameter of the weighting function, and wherein the weighting function is preferably a differential pressure function.

3. Method according to claim 1 or 2, wherein method steps (b) and (c) are iteratively repeated at least one more time.

4. Method according to claim 3, wherein the weighting function applied in the second and/or higher iteration loop differs from the weighting function applied in the first iteration loop.

5. Method according to claim 4, wherein the weighting function applied in the second and/or higher iteration loop is a differential pressure function having the clamp pressure $clamp_n$(t) as an input parameter and having the approximated pulse wave $pulse_{approx}$(t) obtained in the previous iteration loop as another input parameter.

6. Method according to any one of claims 3 to 5, wherein the weighting function applied in the second and/or higher iteration loop is a triangular function, preferably having its maximum when the clamp pressure $clamp_n$(t) equals a predetermined difference, preferably zero, to the approximated pulse wave $pulse_{approx}$(t) obtained in a previous iteration loop.

7. Method according to any one of claims 3 to 5, wherein the weighting function applied in the second and/or higher iteration loop is a bell-shaped function, preferably having its maximum when the clamp pressure $clamp_n$(t) equals a predetermined difference, preferably zero, to the approximated pulse wave $pulse_{approx}$(t) obtained in a previous iteration loop.

8. Method according to any one of claims 4 to 7, wherein the weighting function of the first iteration loop is determined as follows:

$$weight1_n = 1 \quad \text{if} \quad DAP < clamp(t) < SAP,$$

$$weight1_n = 0 \quad \text{otherwise.}$$

9. Method according to any one of claims 3 to 8, wherein method step (c) of the second and/or higher iteration loop further comprises: scaling the approximated pulse wave $pulse_{approx}$(t) to the difference between the diastolic blood pressure value $DAP$ and the systolic blood pressure value $SAP$ of the patient.

10. Method according to any one of the preceding claims, wherein, in method step (a), the clamp pressure $clamp_n$(t) is increased or decreased continuously, preferably at a substantially constant rate.

11. Method according to any one of the preceding claims, wherein method step (a) further comprises: scaling the measured pulse signals $pulse_{n\_measured}(t)$ to the difference between the diastolic blood pressure value $DAP$ and the systolic blood pressure value SAP of the patient.

12. Method according to claim 11, wherein, scaling of the measured pulse signals $pulse_{n\_measured}(t)$ in method step (a) is performed by applying the following formula:

$$pulse_{n\_measured\_scaled}(t) = offset_n + scale_n \times pulse_{n\_measured}(t),$$

wherein the parameter $offset_n$ is preferably calculated as follows:

$$offset_n = DAP - \min\left(pulse_{n\_measured}(t)\right),$$

and wherein the parameter $scale_n$ is preferably calculated as follows:

$$scale_n = \frac{SAP - DAP}{\max\left(pulse_{n\_measured}(t)\right) - \min\left(pulse_{n\_measured}(t)\right)}.$$

13. Logic unit for approximating a patient's pulse wave based on a non-invasive blood pressure measurement, configured to carry out the following steps:

- weighting previously measured pulse signals $pulse_{n\_measured}(t)$ using a weighting function to obtain weighted pulse signals $pulse_{n\_weighted}(t)$, the previously measured pulse signals $pulse_{n\_measured}(t)$ being measured using a not-constant clamp pressure $clamp_n(t)$;
- adding up the weighted pulse signals $pulse_{n\_weighted}(t)$ to obtain an approximation of the patient's pulse wave $pulse_{approx}(t)$,

wherein these steps are preferably iteratively repeated at least one more time.

14. System for approximating a patient's pulse wave based on a non-invasive blood pressure measurement, comprising the logic unit according to claim 13 and a blood pressure measurement device, the blood pressure measurement device being configured for non-invasively measuring a sequence $n=1...N$ of pulse signals of a patient to obtain the measured pulse signals $pulse_{n\_measured}(t)$, wherein the system is configured for providing the measured pulse signals $pulse_{n\_measured}(t)$ as input values to the logic unit.

15. System according to claim 14, wherein the blood pressure measurement device comprises a pressure cuff, the pressure cuff being preferably configured for being disposed around a patient's arm so as to measure the patient's arterial blood pressure in a non-invasive way.

16. System according to claim 15, further comprising a second blood pressure measurement device being adapted for non-invasively measuring peripheral blood pressure waveform data of the patient in a continuous way, wherein the system is adapted to apply a transfer function to reconstruct central blood pressure waveforms from the measured peripheral blood pressure waveform data based on the approximated pulse wave $pulse_{approx}(t)$.

17. System according to claim 16, wherein the approximated pulse wave $pulse_{approx}(t)$ of the patient is determined at substantially regular intervals, and wherein the transfer function is regularly recalibrated based on the regularly determined approximated pulse wave $pulse_{approx}(t)$ of the patient.

**Patentansprüche**

1. Verfahren zum Approximieren einer Pulswelle eines Patienten auf der Basis einer nicht invasiven Blutdruckmessung, das die folgenden Schritte umfasst:

(a) nicht invasives Messen einer Folge $n = 1 ... N$ von Pulssignalen $pulse_{n\_measu\text{-}red}(t)$ eines Patienten, dabei Anlegen eines nicht konstanten Klemmdrucks $clamp_n(t)$;
(b) Gewichten der gemessenen Pulssignale $pulse_{n\_measured}(t)$ unter Verwendung einer Gewichtungsfunktion, um gewichtete Pulssignale $pulse_{n\_measured}(t)$ zu erhalten;
(c) Addieren der gewichteten Pulssignale $pulse_{n\_weighted}(t)$, um eine Approximation der Pulswelle $pulse_{approx}(t)$ des Patienten zu erhalten.

2. Verfahren nach Anspruch 1, wobei der Klemmdruck $clamp_n(t)$ ein Eingangsparameter der Gewichtungsfunktion ist und wobei die Gewichtungsfunktion vorzugsweise eine Differentialdruckfunktion ist.

3. Verfahren nach Anspruch 1 oder 2, wobei die Verfahrensschritte (b) und (c) mindestens ein weiteres Mal iterativ wiederholt werden.

4. Verfahren nach Anspruch 3, wobei sich die Gewichtungsfunktion, die in der zweiten und/oder in einer höheren Iterationsschleife angewandt wird, von der Gewichtungsfunktion, die in der ersten Iterationsschleife angewandt wird, unterscheidet.

5. Verfahren nach Anspruch 4, wobei die Gewichtungsfunktion, die in der zweiten und/oder in einer höheren Iterationsschleife angewandt wird, eine Differentialdruckfunktion ist, die den Klemmdruck $clamp_n(t)$ als einen Eingangsparameter aufweist und die die approximierte Pulswelle $pulse_{approx}(t)$, die in der vorhergehenden Iterationsschleife erhalten wurde, als einen weiteren Eingangsparameter aufweist.

6. Verfahren nach einem der Ansprüche 3 bis 5, wobei die Gewichtungsfunktion, die in der zweiten und/oder in einer höheren Iterationsschleife angewandt wird, eine trianguläre Funktion ist, die vorzugsweise ihr Maximum aufweist, wenn der Klemmdruck $clamp_n(t)$ eine vorgegebene Differenz, vorzugsweise null, zu der approximierten Pulswelle $pulse_{approx}(t)$, die in einer vorhergehenden Iterationsschleife erhalten wurde, aufweist.

7. Verfahren nach einem der Ansprüche 3 bis 5, wobei die Gewichtungsfunktion, die in der zweiten und/oder in einer höheren Iterationsschleife angewandt wird, eine glockenförmige Funktion ist, die vorzugsweise ihr Maximum aufweist, wenn der Klemmdruck $clamp_n(t)$ eine vorgegebene Differenz, vorzugsweise null, zu der approximierten Pulswelle $pulse_{approx}(t)$, die in einer vorhergehenden Iterationsschleife erhalten wurde, aufweist.

8. Verfahren nach einem der Ansprüche 4 bis 7, wobei die Gewichtungsfunktion der ersten Iterationsschleife wie folgt bestimmt wird:

$$weight1_n = 1 \qquad \text{wenn} \qquad DAP < clamp(t) < SAP,$$

$$weight1_n = 0 \qquad \text{sonst.}$$

9. Verfahren nach einem der Ansprüche 3 bis 8, wobei der Verfahrensschritt (c) der zweiten und/oder einer höheren Iterationsschleife ferner Folgendes umfasst: Skalieren der approximierten Pulswelle $pulse_{approx}(t)$ auf die Differenz zwischen dem diastolischen Blutdruckwert $DAP$ und dem systolischen Blutdruckwert $SAP$ des Patienten.

10. Verfahren nach einem der vorhergehenden Ansprüche, wobei im Verfahrensschritt (a) der Klemmdruck $clamp_n(t)$ ununterbrochen und vorzugsweise mit einer im Wesentlichen konstanten Rate erhöht oder erniedrigt wird.

11. Verfahren nach einem der vorhergehenden Ansprüche, wobei der Verfahrensschritt (a) ferner Folgendes umfasst: Skalieren der gemessenen Pulssignale $pulse_{n\_measured}(t)$ auf die Differenz zwischen dem diastolischen Blutdruckwert $DAP$ und dem systolischen Blutdruckwert $SAP$ des Patienten.

12. Verfahren nach Anspruch 11, wobei das Skalieren der gemessenen Pulssignale $pulse_{n\_measured}(t)$ im Verfahrensschritt (a) durch Anwenden der folgenden Formel durchgeführt wird:

$$pulse_{n\_measured\_scaled}(t) = offset_n + scale_n \times pulse_{n\_measured}(t),$$

wobei der Parameter *offset_n* vorzugsweise wie folgt berechnet wird:

$$offset_n = DAP - \min(pulse_{n\_measured}(t)),$$

und

wobei der Parameter *scale_n* vorzugsweise wie folgt berechnet wird:

$$scale_n = \frac{SAP - DAP}{\max(pulse_{n\_measured}(t)) - \min(pulse_{n\_measured}(t))}$$

**13.** Logikeinheit zum Approximieren einer Pulswelle eines Patienten auf der Basis einer nicht invasiven Blutdruckmessung, die konfiguriert ist, die folgenden Schritte auszuführen:

- Gewichten der zuvor gemessenen Pulssignale *pulse_{n_measured}(t)* unter Verwendung einer Gewichtungsfunktion, um die gewichteten Pulssignale *pulse_{n_weighted}(t)* zu erhalten, wobei die zuvor gemessenen Pulssignale *pulse_{n_measured}(t)* unter Verwendung eines nicht konstanten Klemmdrucks *clamp_n(t)* gemessen werden;
- Addieren der gewichteten Pulssignale *pulse_{n_weighted}(t)*, um eine Approximation der Pulswelle *pulse_{approx}(t)* des Patienten zu erhalten,

wobei diese Schritte vorzugsweise mindestens ein weiteres Mal iterativ wiederholt werden.

**14.** System zum Approximieren einer Pulswelle eines Patienten auf der Basis einer nicht invasiven Blutdruckmessung, das die Logikeinheit nach Anspruch 13 und eine Blutdruckmessvorrichtung umfasst, wobei die Blutdruckmessvorrichtung zum nicht invasiven Messen einer Folge *n = 1 ... N* von Pulssignalen eines Patienten konfiguriert ist, um die gemessenen Pulssignale *pulse_{n_measured}(t)* zu erhalten, wobei das System zum Zuführen der gemessenen Pulssignale *pulse_{n_measured}(t)* als Eingangswerte an die Logikeinheit konfiguriert ist.

**15.** System nach Anspruch 14, wobei die Blutdruckmessvorrichtung eine Blutdruckmanschette umfasst, wobei die Blutdruckmanschette vorzugsweise konfiguriert ist, derart um den Arm eines Patienten angeordnet zu werden, dass der arterielle Blutdruck des Patienten auf nicht invasive Weise gemessen wird.

**16.** System nach Anspruch 15, das ferner eine zweite Blutdruckmessvorrichtung umfasst, die zum ununterbrochenen, nicht invasiven Messen der peripheren Blutdruckwellenformdaten des Patienten ausgelegt ist, wobei das System ausgelegt ist, eine Übertragungsfunktion anzuwenden, um auf der Basis der approximierten Pulswelle *pulse_{approx}(t)* die zentralen Blutdruckwellenformen aus den gemessenen, peripheren Blutdruckwellenformdaten zu rekonstruieren.

**17.** System nach Anspruch 16, wobei die approximierte Pulswelle *pulse_{approx}(t)* des Patienten in im Wesentlichen regelmäßigen Intervallen bestimmt wird und wobei die Übertragungsfunktion regelmäßig auf der Basis der regelmäßig bestimmten, approximierten Pulswelle *pulse_{approx}(t)* des Patienten rekalibriert wird.

**Revendications**

**1.** Procédé d'approximation d'une onde d'impulsion du patient sur la base de la mesure de la pression sanguine non invasive, comprenant les étapes suivantes :

(a) mesure non-invasive d'une séquence *n=1...N* de signaux d'impulsion *pulse_{n_measured}(t)* d'un patient, en appliquant une pression de clamp non constante *clamp_n (t)* ;
(b) pondération des signaux d'impulsion mesurés *pulse_{n measured}(t)* en utilisant une fonction de pondération pour obtenir des signaux d'impulsion pondérés *pulse_{n_weighted}(t)* ;
(c) ajout des signaux d'impulsion pondérés *pulse_{n_weighted}(t)* pour obtenir une approximation de l'onde d'impulsion du patient *pulse_{approx}(t)*.

**2.** Procédé selon la revendication 1, dans lequel la pression de clamp *clamp_n(t)* est un paramètre de saisie de la fonction de pondération, et dans lequel la fonction de pondération est de préférence une fonction de pression différentielle.

3. Procédé selon la revendication 1 ou 2, dans lequel les étapes de procédé (b) et (c) sont répétées de façon itérative au moins une fois de plus.

4. Procédé selon la revendication 3, dans lequel la fonction de pondération appliquée dans la seconde boucle d'itération et/ou plus élevée diffère de la fonction de pondération appliquée dans la première boucle d'itération.

5. Procédé selon la revendication 4, dans lequel la fonction de pondération appliquée dans la seconde boucle d'itération et/ou plus élevée est une fonction de pression différentielle ayant la pression de clamp $clamp_n(t)$ en tant que paramètre de saisie et ayant l'onde d'impulsion approximée $pulse_{approx}(t)$ obtenue dans la boucle d'itération précédente en tant qu'autre paramètre de saisie.

6. Procédé selon l'une quelconque des revendications 3 à 5, dans lequel la fonction de pondération appliquée dans la seconde boucle d'itération et/ou plus élevée est une fonction triangulaire, ayant de préférence son maximum lorsque la pression de clamp $clamp_n(t)$ est égale à une différence prédéterminée, de préférence zéro, par rapport à l'onde d'impulsion $pulse_{approx}(t)$ approximée obtenue dans une boucle d'itération précédente.

7. Procédé selon l'une quelconque des revendications 3 à 5, dans lequel la fonction de pondération appliquée dans la seconde boucle d'itération et/ou plus élevée est une fonction en forme de cloche ayant de préférence son maximum lorsque la pression de clamp $clamp_n(t)$ est égale à une différence prédéterminée, de préférence zéro, par rapport à l'onde d'impulsion $pulse_{approx}(t)$ approximée obtenue dans une boucle d'itération précédente.

8. Procédé selon l'une quelconque des revendications 4 à 7, dans lequel la fonction de pondération de la première boucle d'itération est déterminée comme suit :

$$weight1_n = 1 \quad \text{si DAP} < clamp(t) < \text{SAP},$$
$$weight1_n = 0 \quad \text{sinon.}$$

9. Procédé selon l'une quelconque des revendications 3 à 8, dans lequel procédé, l'étape (c) de la seconde boucle d'itération et/ou plus élevée comprend en outre : la mise à l'échelle de l'onde d'impulsion approximée $pulse_{approx}(t)$ par rapport à la différence entre la valeur de pression sanguine diastolique DAT et la valeur de pression sanguine systolique SAP du patient.

10. Procédé selon l'une quelconque des revendications précédentes, dans lequel à l'étape (a) de procédé, la pression de clamp $clamp_n(t)$ est augmentée ou abaissée en continu, de préférence à une vitesse essentiellement constante.

11. Procédé selon l'une quelconque des revendications précédentes, dans lequel procédé l'étape (a) comprend en outre : la mise à l'échelle des signaux d'impulsion $pulse_{n\_measured}(t)$ approximée par rapport à la différence entre la valeur de pression sanguine diastolique DAT et la valeur de pression sanguine systolique SAP du patient.

12. Procédé selon la revendication 11, dans lequel la mise à l'échelle des signaux d'impulsion mesurés $pulse_{n\_measured}(t)$ à l'étape (a) de procédé est effectuée en appliquant la formule suivante :

$$pulse_{n\_measured\_scaled}(t) = offset_n + scale_n \; x$$
$$pulse_{n\_measured}(t),$$

où le paramètre $offset_n$ est calculé de préférence comme suit :

$$offset_n = DAP - \min(pulse_{n\_measured}(t)),$$

et
dans lequel le paramètre $scale_n$ est calculé de préférence comme suit :

$$scale_n = \frac{SAP - DAP}{\max\left(pulse_{n\_measured}(t)\right) - \min\left(pulse_{n\_measured}(t)\right)}.$$

**13.** Unité logique d'approximation d'une onde d'impulsion du patient sur la base de la mesure de la pression sanguine non invasive, configurée pour exécuter les étapes suivantes :

- pondération des signaux d'impulsion mesurés au préalable $pulse_{n\_measured}(t)$ en utilisant une fonction de pondération pour obtenir des signaux d'impulsion pondérés $pulse_{n\_weighted}(t)$, *les* signaux d'impulsion mesurés au préalable $pulse_{n\_measured}(t)$ étant mesurés en appliquant une pression de clamp non constante $clamp_n(t)$ ;
- ajout des signaux d'impulsion pondérés $pulse_{n\_weighted}(t)$ pour obtenir une approximation de l'onde d'impulsion du patient $pulse_{approx}(t)$,

dans lequel ces étapes sont de préférence répétées de façon itérative au moins une fois de plus.

**14.** Système d'approximation d'une onde d'impulsion du patient sur la base de la mesure de la pression sanguine non invasive, comprenant l'unité logique selon la revendication 13 et un dispositif de mesure de la pression sanguine, le dispositif de mesure de la pression sanguine étant configuré pour la mesure non-invasive d'une séquence $n=1...N$ de signaux d'impulsion d'un patient pour obtenir les signaux d'impulsion mesurés $pulse_{n\_measured}(t)$, dans lequel le système est configuré pour fournir les signaux d'impulsion mesurés $pulse_{n\_measured}(t)$ en tant que valeurs de saisie à l'unité logique.

**15.** Système selon la revendication 14, dans lequel le dispositif de mesure de la pression sanguine comprend un brassard compressif, le brassard compressif étant configuré de préférence pour être disposé autour du bras d'un patient de façon à mesurer la pression sanguine artérielle du patient de façon non-invasive.

**16.** Système selon la revendication 15, comprenant en outre un second dispositif de mesure de la pression sanguine adapté pour la mesure non-invasive de données de forme d'onde de pression sanguine périphérique d'un patient de façon continue, dans lequel le système est adapté pour appliquer une fonction de transfert pour reconstruire des formes d'onde de pression sanguine centrale à partir des données de forme d'onde de pression sanguine périphérique mesurées sur la base de l'onde d'impulsion approximée $pulse_{approx}(t)$.

**17.** Système selon la revendication 16, dans lequel l'onde d'impulsion approximée $pulse_{approx}(t)$ du patient est déterminée à intervalles essentiellement réguliers, et dans lequel la fonction de transfert est régulièrement recalibrée sur la base de l'onde d'impulsion approximée $pulse_{approx}(t)$ déterminée régulièrement du patient.

artery

pressure cuff

manometer

Fig. 1

ECG
signal

time

Fig. 2

manometer
signal

SAP

DAP

time

Fig. 3

Fig. 4

EP 2 759 258 B1

Fig. 5

Fig. 6

Fig. 7

```
┌─────────────────────────────┐   ┌─────────────────────────────┐
│ Intermittent central blood  │   │ Continuous peripheral blood │
│ pressure curve p_c(t)       │   │ pressure signal p_p(t)      │
└─────────────────────────────┘   └─────────────────────────────┘
              │                                 │
              ▼                                 ▼
┌─────────────────────────────┐   ┌─────────────────────────────┐
│          P_c(f)             │   │          P_p(f)             │
└─────────────────────────────┘   └─────────────────────────────┘
                  ╲                     ╱
                   ╲                   ╱
                    ▼                 ▼
          ┌─────────────────────────────┐
          │      Transfer function      │
          │   G(f) = P_c(f)/P_p(f)      │
          └─────────────────────────────┘
                        │
                        ▼
          ┌─────────────────────────────┐
          │   P_c(f)* = G(f)*P_p(f)      │
          └─────────────────────────────┘
                        │
                        ▼
          ┌─────────────────────────────┐
          │   Continuous calibrated      │
          │ blood pressure signal p_c(t)*│
          └─────────────────────────────┘
```

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6503206 B1
- US 20040002659 A1

- EP 0078090 A1